# EUROPEAN PATENT SPECIFICATION

(11) **EP 1 051 538 B1**
(45) Date of publication and mention of the grant of the patent: **22.10.2003**
(21) Application number: 99907438.8
(22) Date of filing: 28.01.1999
(51) Int. Cl.: C23C 22/24, A61B 17/06

(54) **PROCESS FOR THE MANUFACTURE OF BLACKENED SURGICAL NEEDLES**
VERFAHREN ZUR HERSTELLUNG GESCHWÄRZTER CHIRURGISCHER NADELN
PROCEDE DE FABRICATION D'AIGUILLES CHIRURGICALES NOIRCIES

(30) Priority: 30.01.1998 DE 19804846
(43) Date of publication of application: 15.11.2000
(73) Proprietor: Ethicon GmbH, 22851 Norderstedt (DE)
(72) Inventor: HINSCH, Bernhard, D-22851 Norderstedt (DE); HÜNDORF, Uwe, D-24558 Henstedt-Ulzburg (DE); RUTHENBERG, Jürgen, D-22175 Hamburg (DE)
(74) Representative: UEXKÜLL & STOLBERG
(86) International application number: EP9900540
(87) International publication number: WO99039024

(56) References cited:
- EP-A- 0 052 056
- US-A- 2 283 171
- US-A- 2 312 066
- US-A- 3 210 220
- US-A- 4 959 068

## Description

The invention relates to a process for the manufacture of blackened surgical needles.

Surgical needles normally have a metallic, polished surface. In the case of some applications, however, in particular in heart surgery and in microsurgery, the reflexes caused by such needles are a problem during an operation. Therefore, surgical needles with a blackened surface have been developed in order to avoid this problem. "Blackened" surface is to be understood below to mean generally a dark surface which does not reflect light in an inconvenient manner and which does not necessarily have to be black (i.e. so dark that it no longer reflects any light at all).

A process is known from US-A-4 959 068 for the manufacture of blackened surgical needles, in which unfinished needles produced from a chromium-containing steel are dipped into a solution of approximately 54 to 61 wt.% sulphuric acid and approximately 5.6 to 9.1 wt.% potassium dichromate in water, which solution has a temperature greater than 100°C. At a treatment temperature of 110°C to 130°C, the originally polished unfinished needles have a sufficiently blackened surface after approximately 4 minutes to 30 minutes.

US-A-2 312 066 discloses a method of coloring chromium-containing stainless steel, in which the specimen to be treated is immersed in a solution consisting of 36-50 parts by weight sulphuric acid (1.84 sp. g.), 30-50 parts by weight water and 10-14 parts by weight etching inhibitor, which is preferably a dichromate. Treatment temperatures lie between Zoom temperature and the boiling point. In US-A- 2 283 171, it is stated that treatments at low temperatures in such solutions are commercially unpractical because of the long treatment times.

Blackened surgical needles have the disadvantage that the force necessary to draw the needle through tissue (force of penetration) is greater than in the case of a conventional needle with a polished surface. This is also the case if the surgical needle is siliconized after blackening, i.e. is provided with a silicone-containing surface coating.

It is the object of the invention to provide a process for the manufacture of blackened surgical needles which can be carried out in a cost-favourable and easily controllable manner and which delivers surgical needles with a blackened surface which have more favourable penetration properties than blackened surgical needles manufactured according to conventional processes.

This object is achieved by a process for the manufacture of blackened surgical needles having the features of Claim 1. Advantageous versions are given in the dependent claims.

In the process according to the invention for the manufacture of blackened surgical needles, unfinished needles produced from a chromium-containing steel are dipped into an acidic aqueous solution with a chromium(VI) compound, which solution has a temperature in the range from 10°C to 40°C, preferably in the range from 18°C to 25°C.

It has surprisingly been shown that, despite it being expressly mentioned in US patent No. 4 959 068 that the treatment temperature on blackening must be above 100°C, blackened surgical needles can be obtained when the treatment temperature is only in the range from 10°C to 40°C. The treatment in the acidic aqueous solution with a chromium(VI) compound preferably takes place at room temperature, i.e. in the range from 18°C to 25°C. Blackened surgical needles manufactured in this way have a firmly adhering surface. After they are siliconized, the forces of penetration necessary to draw the needles through a specimen are less than those of blackened surgical needles in which the blackening treatment was carried out at a higher temperature. A further advantage of the process according to the invention is that it can be optimally adapted to the batch of unfinished needles to be treated. Since it is necessary in the case of the low treatment temperature to dip the needles into the acidic aqueous solution for at least 15 hours, it is possible in individual cases to define the exact point in time when the unfinished needles of the batch to be treated are sufficiently blackened and the treatment in the acidic aqueous solution can be ended.

The chromium(VI) compound is preferably chromium(VI) oxide. In the case of a preferred embodiment of the process according to the invention, the acidic aqueous solution contains 10 to 20 wt.% chromium(VI) oxide. This chromium content is clearly higher than in the case of the process known from US patent No. 4 959 068. The higher chromium content has a favourable effect on the frictional resistance of the surgical needles, which presumably is due to the fact that the high chromium content reduces the danger of pitting. A blackened surgical needle manufactured in this way and provided with a silicone coating therefore has a particularly smooth surface, for which reason its force of penetration is lower than in the case of a needle treated in a solution with a low chromium content.

In a preferred embodiment, the acidic aqueous solution contains sulphuric acid, preferably 20 to 40 wt.%. In an embodiment, the acidic aqueous solution contains 15 to 19 wt.% chromium(VI) oxide and 28 to 35 wt.% sulphuric acid.

The unfinished needles are dipped into the acidic aqueous solution for a period in the range from 15 hours to 150 hours. The optimum period depends on the material of the unfinished needles and on the composition and temperature of the acidic aqueous solution. The aim is to manufacture surgical needles with a sufficiently blackened surface which have as low forces of penetration as possible. Therefore, the surgical needles should not be left too long in the acidic aqueous solution. In general, it is the case that, the higher the temperature of the acidic aqueous solution, the smaller the necessary period. The period which produces the best results can be determined in individual cases by trials.

Unfinished needles which are produced from a high-grade steel with the material number 4031 (chromium content ca. 13.0 to 13.5 %) are preferably dipped into the acidic aqueous solution for a period of 60 hours to 150 hours.

Unfinished needles which are produced from a high-grade steel with the material number 4310 (chromium content ca. 16.0 % to 18.0 %) are preferably dipped into the acidic aqueous solution for a period of 20 hours to 50 hours.

Unfinished needles which are produced from a steel with the material number 1.4456 (chromium content ca. 16.0 % to 20.0 %) are preferably dipped into the acidic aqueous solution for a period of 20 hours to 70 hours.

In a preferred embodiment of the process according to the invention, the unfinished needles are polished before being dipped into the acidic aqueous solution, so that they have a smooth and clean surface on which the acidic aqueous solution can act in order to bring about the desired blackening.

The blackened needles are preferably rinsed with water after removal from the acidic aqueous solution in order to end the action of the acid. After rinsing with water, the blackened needles can be placed into an aqueous solution of a wetting agent, which solution is preferably degassed with the blackened needles lying therein at below-atmospheric pressure. These steps can optionally be repeated. The blackened needles can then be rinsed again with water.

In a preferred embodiment, the blackened needles are finally rinsed in alcohol and dried at a temperature above room temperature.

As already mentioned, a "blackened" surgical needle is not necessarily to be understood to mean a surgical needle with a deep-black surface, but the term "blackened" is here to be understood to mean generally that a blackened surgical needle has a dark surface which does not cause any disturbing reflexes.

The invention is described in more detail below with reference to embodiments.

### Example 1

Unfinished needles with a ready-ground tip and polished surface were manufactured from stainless steel wire with the material number 4031 YC (equivalent to the US material number 420), from wires with diameters of 0.74 mm to 1.78 mm. The chromium content of the steel used was in the range from 13.0 % to 13.5 %. The unfinished needles were not siliconized.

In order to blacken the unfinished needles, an acidic aqueous solution called "blackening solution" below was prepared by mixing 3000 ml demineralized water with 1000 ml concentrated sulphuric acid and dissolving 1000 g chromium(VI) oxide therein.

A batch of unfinished needles to be blackened of a given needle type was dipped into the blackening solution so that the unfinished needles were covered by at least 1 cm of the blackening solution. The vessel used for this purpose was situated under a hood. The treatment in the blackening solution took place at room temperature, i.e. in the range from 18°C to 25°C.

The period of time for which the unfinished needles were dipped into the blackening solution depended on the measured room temperature, taking as a basis the guide values given in the following table:

| Temperature [°C] | Period [h] |
|---|---|
| 18.0 | 128 |
| 18.5 | 125 |
| 19.0 | 121 |
| 19.5 | 118 |
| 20.0 | 114 |
| 20.5 | 111 |
| 21.0 | 107 |
| 21.5 | 104 |
| 22.0 | 100 |
| 22.5 | 97 |
| 23.0 | 93 |
| 23.5 | 90 |
| 24.0 | 86 |
| 24.5 | 83 |
| 25.0 | 79 |

A few hours before the expiry of the guide value for the period of time allocated to the measured room temperature, some needles were removed from the blackening solution using a magnetic rod and thoroughly rinsed with water. Their colour was then compared on a black background with the colour standard of a needle catalogue which contained needles of the given needle type optimally blackened according to the described process. If the colouring was not yet sufficient, the needles were again placed back into the vessel with the blackening solution. Depending on the progress of the blackening procedure, the needles were checked as described above every 0.5 to 2 hours.

Once blackening was complete, the blackening solution was poured out and the blackened needles were pre-rinsed with water until the rinsing water no longer had a visible yellow colouring. The blackened needles were then subjected to the washing procedure described below.

To this end, a solution of 0.06 % Triton X 100 in water was prepared. Triton X 100 is the brand name of a wetting agent which is marketed by Merck in 6% aqueous solution. Its chemical name is O-[4-(1,1,3,3-tetramethylbutyl)-phenyl]-deca(oxy-ethylene).

The blackened needles pre-rinsed in the manner described previously were covered with the Triton solution in a vessel. The vessel was placed into a desiccator, and the desiccator was evacuated for at least 10 minutes using a vacuum pump in order to make possible a degassing of the Triton solution with the blackened needles lying therein. The desiccator was then aerated, the Triton solution was poured out and replaced by a fresh Triton solution. The fresh Triton solution with the blackened needles lying therein was degassed again in the desiccator for at least 10 minutes.

After this degassing procedure, the pH values of the Triton solution after the second degassing and of a fresh Triton solution were determined. If the pH value of the Triton solution after the second degassing was more than 0.2 pH units less than that of the fresh Triton solution, the Triton solution was poured out of the vessel and again replaced by a fresh Triton solution in order to repeat the degassing process in the desiccator.

The blackened needles in the vessel used were then rinsed with water until no more foam formed. The needles were covered with water and again degassed in the desiccator for at least 10 minutes. The water was then poured out. The needles were finally rinsed in isopropanol and then dried in the drying cabinet at 100°C to 120°C for at least 1 hour.

In subsequent process steps, the blackened needles were provided with a silicone coating, packed and sterilized.

### Example 2

Unfinished needles with a ready-ground tip and polished surface were manufactured from stainless steel wire with the material numbers 4310 FB (0.16 mm to 0.36 mm diameter) and 4310 FB Streduc (0.46 mm to 0.66 mm diameter). This steel had a chromium content in the range from 16.0 % to 18.0 %. The surfaces of the unfinished needles were not siliconized.

The unfinished needles of a batch with a given needle type to be subjected to the blackening treatment were dipped into the blackening solution prepared according to Example 1, being covered to at least 1 cm by the blackening solution. The vessel with the unfinished needles and the blackening solution was situated under a hood.

The period of time for the treatment of the needles in the blackening solution was of the order of magnitude of 24 to 30 hours in this example. After approximately 24 hours had elapsed, first sample needles were removed using a magnetic rod and were rinsed thoroughly with water in order to compare their colour on a black background with the colour standards of a needle catalogue made analogously to Example 1. If the colouring or blackening was not yet sufficient, the needles were put back again into the vessel. Depending on the progress of the blackening process, the needles were checked in the manner previously described every 0.5 to 2 hours.

After blackening of the needles was complete, the blackening solution was poured out. The blackened needles were then rinsed with water until the rinsing water no longer had a yellow colouring.

The blackened needles were then washed in a Triton solution, rinsed with water, rinsed with isopropanol and dried in the drying cabinet at 100°C to 120°C for at least 1 hour, as described in Example 1.

### Example 3

Unfinished needles with a ready-ground tip and polished surface were manufactured from wire with the material number 1.4456 CA, a relatively new austenitic iron material with at most 0.1 % carbon, at most 1.0 % silicon, 16.0 % to 20.0 % manganese, 16.0 % to 20.0 % chromium, at most 0.3 % nickel, 1.8 % to 2.5 % molybdenum and 0.7 % to 1.0 % nitrogen. The surfaces of the unfinished needles were not siliconized.

The unfinished needles of a batch with a given needle type to be subjected to the blackening treatment were dipped into the blackening solution prepared according to Example 1. The blackening treatment and the after-treatment were carried out in a similar manner to that described in Examples 1 and 2, the period of time for the treatment, carried out at room temperature, of the needles in the blackening solution being in the range from 20 hours to 70 hours.

Experiments showed that, after siliconization, the forces of penetration of blackened needles according to Example 3 are comparable with the values for needles according to Example 1 and Example 2.

### Example 4

The force of penetration as a function of the temperature of the blackening solution and of the period of time for the treatment in the blackening solution was investigated for blackened needles of stainless steel wire with the material number 4031 YC (see Example 1).

To this end, unfinished needles in the form of cutting needles (3/8 circle) of 0.81 mm diameter with a ready-ground tip were dipped into the blackening solution according to Example 1 and treated, after-treated and siliconized in a manner similar to that described in Example 1.

In a first series of tests, blackening solutions at two different temperatures, namely room temperature (21.5°C) and 30°C, were used. The forces of penetration of the blackened and siliconized needles were then determined under standard conditions using polyurethane foam. An average value of 228 g resulted for the force of penetration of the needles treated at room temperature and an average value of 278 g, i.e. a noticeably higher value, resulted for the needles treated at 30°C.

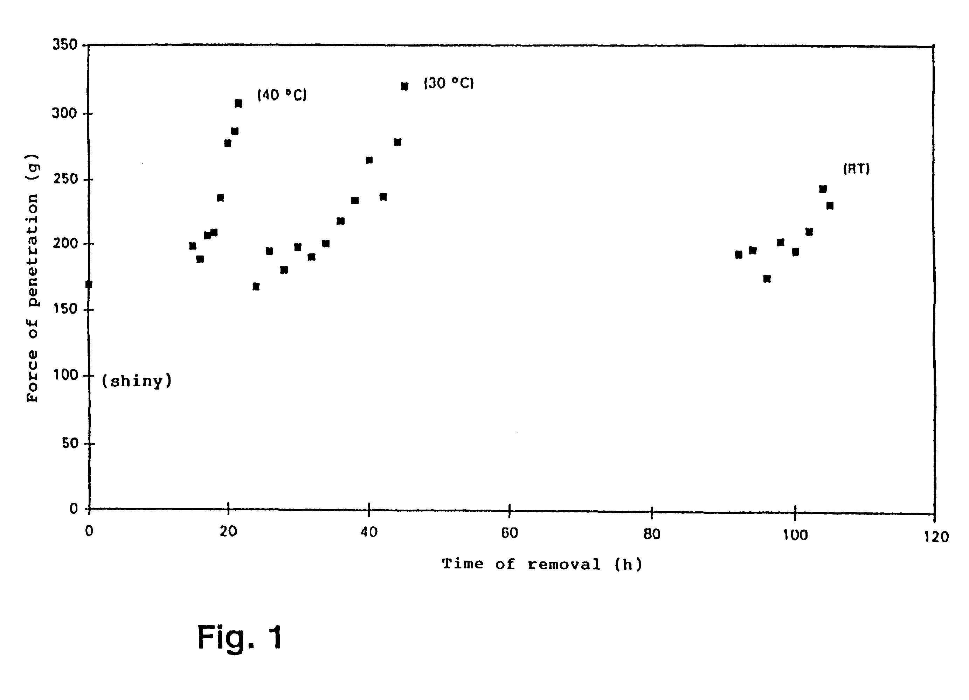

In a second series of tests, in contrast to Example 1, the colouring of the needles was not continuously monitored in order to find an optimum period of time for the treatment in the blackening solution, but the treatment took place for preset fixed periods of time after the expiry of which respective sample needles were removed from the blackening solution. Blackening solutions at room temperature (RT), 30°C and 40°C were used. Figure 1 shows the results of the measurements of the force of penetration finally carried out on the blackened and siliconized needles. Each point corresponds to the average for several needles treated under the same conditions.

Figure 1 clearly shows how, for a given temperature of the blackening solution, the force of penetration increases with an increasing residence time in the blackening solution. Furthermore, it becomes clear that a lower temperature of the blackening solution requires a longer period of time for the treatment in the blackening solution. Finally, the trend can also be seen that a higher temperature of the blackening solution results in a greater force of penetration. On the very left in Figure 1 (time of removal = 0 h), the force of penetration for unblackened (shiny) needles is shown which is only slightly less than for needles blackened according to the invention.

## Claims

1. Process for the manufacture of blackened surgical needles, in which unfinished needles produced from a chromium-containing steel are dipped into an acidic aqueous solution containing sulphuric acid and a chromium(VI) compound, **characterized in that** the acidic aqueous solution has a temperature in the range from 10°C to 40°C and **in that** the unfinished needles are dipped into the acidic aqueous solution for a period in the range from 15 hours to 150 hours.

2. Process according to Claim 1, **characterized in that** the acidic aqueous solution has a temperature in the range from 18°C to 25°C.

3. Process according to Claim 1 or 2, **characterized in that** the chromium(VI) compound is chromium(VI) oxide.

4. Process according to Claim 3, **characterized in that** the acidic aqueous solution contains 10 to 20 wt.% chromium(VI) oxide.

5. Process according to one of Claims 1 to 4, **characterized in that** the acidic aqueous solution contains 20 to 40 wt.% sulphuric acid.

6. Process according to Claim 1 or 2, **characterized in that** the acidic aqueous solution contains 15 to 19 wt.% chromium(VI) oxide and 28 to 35 wt.% sulphuric acid.

7. Process according to one of the preceding claims, **characterized in that** the unfinished needles are polished before being dipped into the acidic aqueous solution.

8. Process according to one of the preceding claims, **characterized in that** the blackened needles are rinsed with water after removal from the acidic aqueous solution.

9. Process according to Claim 8, **characterized in that**, after rinsing with water, the blackened needles are placed into an aqueous solution of a wetting agent, which solution is preferably degassed with the blackened needles lying therein at below-atmospheric pressure, these steps optionally being repeated.

10. Process according to Claim 9, **characterized in that** the blackened needles are subsequently rinsed with water.

11. Process according to one of Claims 8 to 10, **characterized in that** the blackened needles are finally rinsed in alcohol and dried at a temperature above room temperature.

## Patentansprüche

1. Verfahren zum Herstellen von geschwärzten chirurgischen Nadeln, bei dem aus einem chromhaltigen Stahl gefertigte Rohnadeln in eine saure wäßrige Lösung getaucht werden, die Schwefelsäure und eine Chrom(VI)-Verbindung enthält, **dadurch gekennzeichnet, daß** die saure wäßrige Lösung eine Temperatur im Bereich von 10°C bis 40°C hat und daß die Rohnadeln für eine Zeitdauer im Bereich von 15 Stunden bis 150 Stunden in die saure wäßrige Lösung getaucht werden.

2. Verfahren nach Anspruch 1, **dadurch gekennzeichnet, daß** die saure wäßrige Lösung eine Temperatur im Bereich von 18°C bis 25°C hat.

3. Verfahren nach Anspruch 1 oder 2, **dadurch gekennzeichnet, daß** die Chrom(VI)-Verbindung Chrom(VI)oxid ist.

4. Verfahren nach Anspruch 3, **dadurch gekennzeichnet, daß** die saure wäßrige Lösung 10 bis 20 Gew.-% Chrom(VI)oxid enthält.

5. Verfahren nach einem der Ansprüche 1 bis 4, **dadurch gekennzeichnet, daß** die saure wäßrige Lösung 20 bis 40 Gew.-% Schwefelsäure enthält.

6. Verfahren nach Anspruch 1 oder 2, **dadurch gekennzeichnet, daß** die saure wäßrige Lösung 15 bis 19 Gew.-% Chrom(VI)oxid und 28 bis 35 Gew.-% Schwefelsäure enthält.

7. Verfahren nach einem der vorhergehenden Ansprüche, **dadurch gekennzeichnet, daß** die Rohnadeln vor dem Eintauchen in die saure wäßrige Lösung poliert werden.

8. Verfahren nach einem der vorhergehenden Ansprüche, **dadurch gekennzeichnet, daß** die geschwärzten Nadeln nach dem 'Herausnehmen aus der sauren wäßrigen Lösung mit Wasser gespült werden.

9. Verfahren nach Anspruch 8, **dadurch gekennzeichnet, daß** die geschwärzten Nadeln nach dem Spülen mit Wasser in eine wäßrige Lösung eines Benetzungsmittels gelegt werden, die vorzugsweise mit den darin liegenden geschwärzten Nadeln unter Unterdruck entgast wird, wobei diese Schritte optional wiederholt werden.

10. Verfahren nach Anspruch 9, **dadurch gekennzeichnet, daß** die geschwärzten Nadeln anschließend mit Wasser gespült werden.

11. Verfahren nach einem der Ansprüche 8 bis 10, **dadurch gekennzeichnet, daß** die geschwärzten Nadeln abschließend in Alkohol gespült und bei einer oberhalb der Raumtemperatur liegenden Temperatur getrocknet werden.

## Revendications

1. Procédé pour la fabrication d'aiguilles chirurgicales noircies dans lequel les aiguilles non-finies produites à partir d'acier contenant du chrome sont plongées dans une solution aqueuse acide contenant de l'acide sulfurique et un chrome hexavalent(VI), **caractérisé en ce que** la solution aqueuse acide est à une température comprise dans la plage allant de 10°C à 40°C et **en ce que** les aiguilles non-finies sont plongées dans une solution aqueuse acide pendant une période comprise dans la plage allant de 15 à 150 heures.

2. Procédé selon la revendication 1, **caractérisé en ce que** la solution aqueuse acide est à une température comprise dans la plage allant de 18°C à 25°C.

3. Procédé selon la revendication 1 ou 2, **caractérisé en ce que** le chrome hexavalent (VI) est un oxyde chromique(VI).

4. Procédé selon la revendication 3, **caractérisé en ce que** la solution aqueuse acide contient de 10 à 20% en poids d'oxyde chromique(VI).

5. Procédé selon l'une des revendications 1 à 4, **caractérisé en ce que** la solution aqueuse acide contient de 20 à 40% en poids d'acide sulfurique.

6. Procédé selon la revendication 1 ou 2, **caractérisé en ce que** la solution aqueuse acide contient de 15 à 19% en poids d'oxyde chromique (VI) et de 28 à 35% en poids d'acide sulfurique.

7. Procédé selon l'une des revendications précédentes, **caractérisé en ce que** les aiguilles non-finies sont polies avant d'être plongées dans la solution aqueuse acide.

8. Procédé selon l'une des revendications précédentes, **caractérisé en ce que** les aiguilles noircies sont rincées à l'eau après être retirées de la solution aqueuse acide.

9. Procédé selon la revendication 8, **caractérisé en ce que**, après rinçage à l'eau, les aiguilles noircies sont placées dans une solution aqueuse d'un agent mouillant, laquelle solution est de préférence dégazée avec les aiguilles noircies immergées dans celle-ci à une pression inférieure à la pression atmosphérique, ces étapes pouvant facultativement être répétées.

10. Procédé selon la revendication 9, **caractérisé en ce que** les aiguilles noircies sont ensuite rincées à l'eau.

11. Procédé selon l'une des revendications 8 à 10, **caractérisé en ce que** les aiguilles noircies sont finalement rincées à l'alcool et séchées à une température supérieure à la température ambiante.
